# EUROPEAN PATENT APPLICATION

(11) **EP 4 729 522 A1**
(43) Date of publication of application: **22.04.2026**
(21) Application number: 24825207.4
(22) Date of filing: 17.06.2024
(51) Int. Cl.: C07D 498/20, A61K 31/537, A61P 31/18

(54) **CRYSTAL FORMS OF SODIUM SALT OF POLYCYCLIC CARBAMOYLPYRIDONE DERIVATIVE, AND PREPARATION METHOD THEREFOR AND USE THEREOF**

(30) Priority: 19.06.2023 CN 202310727199
(71) Applicant: Jiangsu Aidea Pharmaceutical Group Co., Ltd., Yangzhou, Jiangsu 225123 (CN); Yangzhou Aidea Pharmaceutical Technology Co., Ltd., Yangzhou, Jiangsu 225127 (CN); Nanjing Aidea Pharmaceutical Technology Co., Ltd., Nanjing, Jiangsu 210044 (CN)
(72) Inventor: TIAN, Zongyong, Yangzhou, Jiangsu 225123 (CN); HU, Tianjin, Yangzhou, Jiangsu 225123 (CN); WEI, Yufeng, Yangzhou, Jiangsu 225123 (CN); ZHANG, Li, Yangzhou, Jiangsu 225123 (CN); YUN, Xinming, Yangzhou, Jiangsu 225123 (CN); TANG, Tingting, Yangzhou, Jiangsu 225123 (CN); QI, Hong, Yangzhou, Jiangsu 225123 (CN); FU, Heliang, Yangzhou, Jiangsu 225123 (CN)
(74) Representative: Ipsilon
(86) International application number: PCT/CN2024/099647
(87) International publication number: WO 2024/260312

(57) **Abstract**

Provided in the present invention are crystal forms of the sodium salt of an HIV-1 integrase inhibitor molecule. Specifically, provided in the invention are crystal form A and crystal form B of the sodium salt of a compound as shown in formula 1. The crystal forms of the present invention can be used for preventing and/or treating diseases related to HIV infections and selectively inhibiting the activity of HIV integrase.

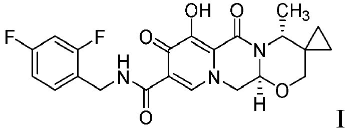

## Description

### Technical field

The present invention relates to the field of pharmaceutical chemistry, specifically to crystal forms of sodium salt of polycyclic carbamoylpyridone derivative, and preparation method therefor and use thereof.

### Background

HIV is still a chronic infection, and the number of new HIV infections increases year by year. There is no protein in a host cell that has a structure similar to HIV-1 integrase, and inhibiting HIV-1 integrase activity will not affect the normal function of the host cell. Integrase inhibitors block the replication process of HIV by inhibiting the strand transfer reaction, and thus they are also known as integrase strand transfer inhibitors. An American observational study published in J Acquir Immune Defic Syndr 2020; 84: 396-399 showed that people on integrase inhibitor-based antiretroviral therapy had a 21% reduced risk of serious cardiovascular events compared to those on alternative medications. Due to their excellent antiviral efficacy, high barrier to resistance, and favorable safety profile, they have become the core drugs for current antiviral therapy and are the first-line recommendation in international AIDS treatment guidelines.

In recent years, as research on the structure of integrase and its inhibitors has deepened, an increasing number of highly effective and low-toxicity integrase inhibitors have emerged. However, drug-resistant strains of integrase inhibitors (such as Y143C, Q148H, and N155H) have subsequently appeared. The problem of drug resistance of virus remains unresolved, making it particularly important to develop new integrase inhibitors that are highly effective, low-toxic, and active against resistant strains.

Therefore, it is of great significance to develop a highly stable and readily druggable integrase inhibitor.

### Summary of the invention

One objective of the present invention is to provide a highly stable sodium salt crystal form A and sodium salt crystal form B of a HIV integrase inhibitor molecule having a structure of formula I.

Another objective of the present invention is to provide a method for preparing sodium salt crystal form A of a compound of Formula I and uses thereof, and a method for preparing sodium salt crystal form B of a compound of Formula I and uses thereof.

In the first aspect of the present invention, provided is a sodium salt crystal form of a compound of formula I, 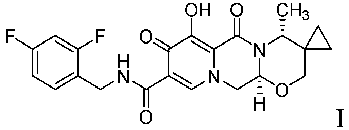
the sodium salt crystal form of the compound of formula I is sodium salt crystal form A, and the sodium salt crystal form A has the following X-ray powder diffraction characteristic peaks (2 θ angle): 15.587 °± 0.2 °, 9.107 °± 0.2 °, 24.121 °± 0.2 °, 7.806 °± 0.2 °, 20.828 °± 0.2 °; or
the sodium salt crystal form of the compound of formula I is sodium salt crystal form B, and the sodium salt crystal form B has the following X-ray powder diffraction characteristic peaks (2 θ angle): 15.384 °± 0.2 °, 25.98 °± 0.2 °, 19.097 °± 0.2 °, 8.673 °± 0.2 °, 21.609 °± 0.2 °.

In another preferred embodiment, the X-ray powder diffraction pattern of the sodium salt crystal form A further includes 2 or more (3, 4, or 5) X-ray powder diffraction characteristic peaks (2 θ angles) selected from the group consisting of: 27.738 °± 0.2 °, 22.198 °± 0.2 °, 18.226 °± 0.2 °, 26.147 °± 0.2 °, 18.622 °± 0.2 °, 23.448 °± 0.2 °; or
the X-ray powder diffraction pattern of the sodium salt crystal form B further includes 2 or more (3, 4, or 5) X-ray powder diffraction characteristic peaks (2 θ angles) selected from the group consisting of: 21.115 °± 0.2 °, 18.606 °± 0.2 °, 27.442 °± 0.2 °, 27.18 °± 0.2 °, 17.468 °± 0.2 °, 18.098 °± 0.2 °.

In another preferred embodiment, the sodium salt crystal form A further has one or more X-ray powder diffraction characteristic peaks (2 θ angles) selected from the group consisting of: 16.332 °± 0.2 °, 25.756 °± 0.2 °, 13.420 °± 0.2 °, 17.994 °± 0.2 °, 12.535 °± 0.2 °, 19.299 °± 0.2 °, 19.729 °± 0.2 °, 25.241 °± 0.2 °, 23.712 °± 0.2 °; or
the sodium salt crystal form B further has one or more X-ray powder diffraction characteristic peaks (2 θ angles) selected from the group consisting of: 6.322 °± 0.2 °, 30.947 °± 0.2 °, 17.267 °± 0.2 °, 10.769 °± 0.2 °, 14.046 °± 0.2 °, 29.323 °± 0.2 °, 23.132 °± 0.2 °, 25.18 °± 0.2 °, 33.5 °± 0.2 °.

In another preferred embodiment, the sodium salt crystal form A has X-ray powder diffraction characteristic peaks (2 θ angles) selected from the group consisting of: 22.016 °± 0.2 °, 19.525 °± 0.2 °, 28.184 °± 0.2 °, 11.069 °± 0.2 °, 11.686 °± 0.2 °, 30.882 °± 0.2 °, 16.849 °± 0.2 °, 28.907 °± 0.2 °, 33.217 °± 0.2 °, 21.052 °± 0.2 °, 34.521 °± 0.2 °, 36.875 °± 0.2 °, 13.120 °± 0.2 °, 14.807 °± 0.2 °, 26.963 °± 0.2 °, 30.262 °± 0.2 °, 29.763 ° ± 0.2 °, 31.392 °± 0.2 °, 31.776 °± 0.2 °, 24.425 °± 0.2 °, 38.225 °± 0.2 °, 22.472 °± 0.2 °, 34.820 °± 0.2 °, 39.689 °± 0.2 °, 24.739 °± 0.2 °, 37.645 °± 0.2 °, 35.908 °± 0.2 °, 39.401 °± 0.2 °, 6.308 °± 0.2 °, 35.090 °± 0.2 °, 39.014 °± 0.2 °; or
the sodium salt crystal form B has X-ray powder diffraction characteristic peaks (2 θ angles) selected from the group consisting of: 37.29 °± 0.2 °, 13.481 °± 0.2 °, 38.788 °± 0.2 °, 23.716 °± 0.2 °, 22.543 °± 0.2 °, 35.05 °± 0.2 °, 12.328 °± 0.2 °, 35.49 °± 0.2 °, 30.64 °± 0.2 °, 36.322 °± 0.2 °, 31.99 °± 0.2 °, 12.672 °± 0.2 °, 29.62 °± 0.2 °, 17.07 °± 0.2 °, 36.75 °± 0.2 °.

In another preferred embodiment, the thermogravimetric analysis (TGA) thermogram of the sodium salt crystal form A shows a weight loss of 0.2%~2.0% in the range of 100 °C~380 °C; the differential scanning calorimetry of the sodium salt crystal form A shows an endothermic peak at 385 °C~395 °C.

In another preferred embodiment, the thermogravimetric analysis (TGA) thermogram of the sodium salt crystal form B shows a weight loss of 1.0%~3.0% in the range of 100 °C~368 °C; the differential scanning calorimetry of the sodium salt crystal form B shows an endothermic peak at 385 °C~390 °C.

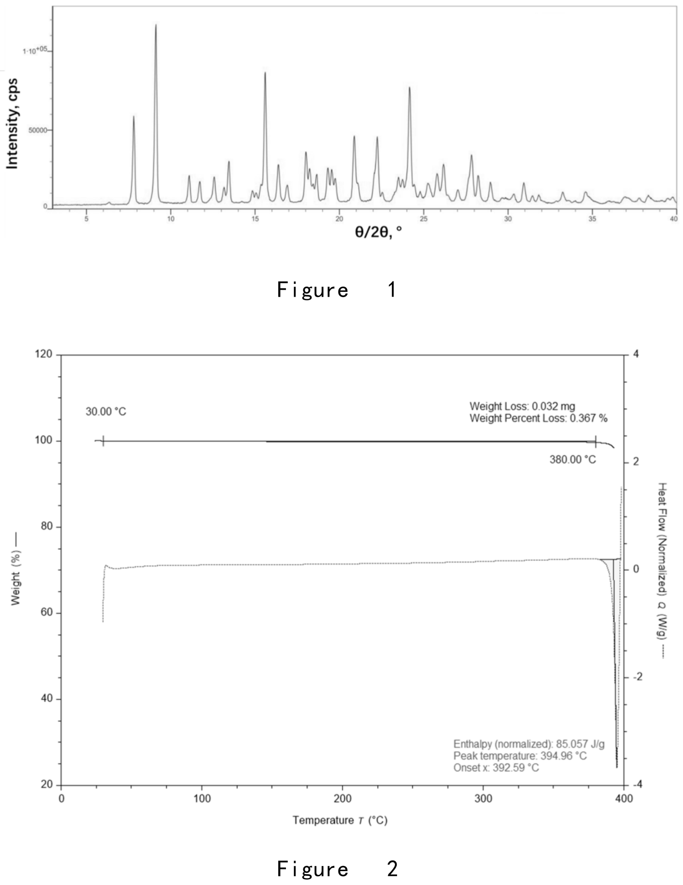

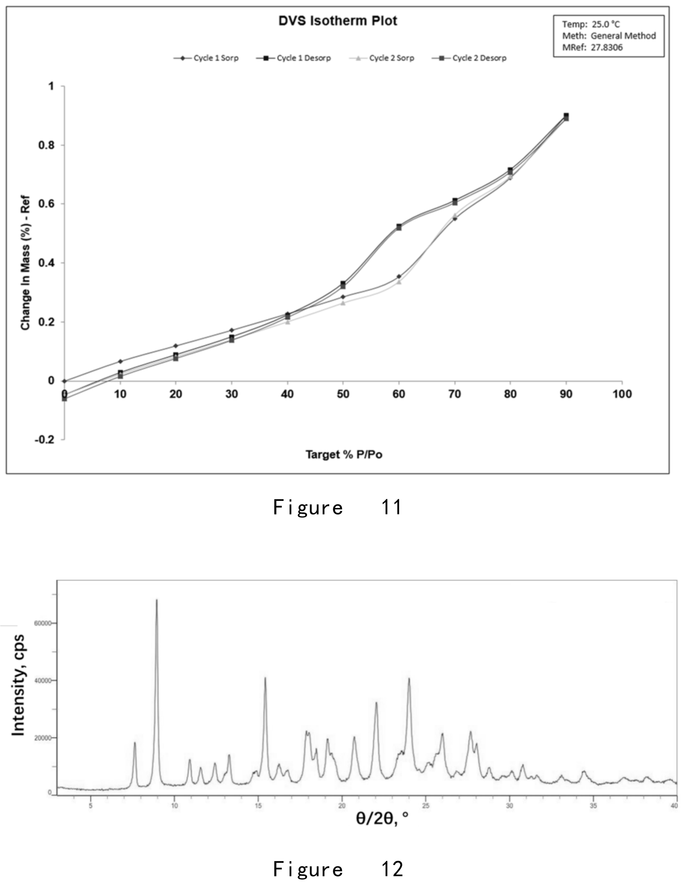

In another preferred embodiment, the sodium salt crystal form A further has one or more of the following characteristics (1)-(4):
(1) The sodium salt crystal form A has XRPD data substantially as shown in Table 1; and/or
(2) The sodium salt crystal form A has an XRPD spectrum substantially as shown in Figure 1; and/or
(3) The sodium salt crystal form A has a DSC&TGA spectrum substantially as shown in Figure 2; and/or
(4) The sodium salt crystal form A has a DVS spectrum substantially as shown in Figure 11.

In the second aspect of the present invention, provided is a method for preparing the crystal form as described in the first aspect of the present invention, wherein the crystal form is a sodium salt crystal form, and the preparation method comprises the steps of:
adding the compound of formula I to an organic solvent or an aqueous organic solvent, reacting with sodium hydroxide, and obtaining the sodium salt crystal form by crystallization.

In another preferred embodiment, the sodium hydroxide is added in the form of an aqueous sodium hydroxide solution, or solid sodium hydroxide.

In another preferred embodiment, the reaction time is 8-30 hours.

In another preferred embodiment, the crystallization time is 2-8 hours, preferably 3-6 hours.

In another preferred embodiment, the crystallization is selected from the group consisting of: crystallization at room temperature, cooling crystallization, solvent evaporation crystallization, seed-induced crystallization, or a combination thereof.

In another preferred embodiment, the cooling crystallization refers to crystallization at -5~25 °C.

In another preferred embodiment, the method further comprises the steps of:
after crystallization, obtaining the sodium salt crystal form by filtering and drying.

In another preferred embodiment, the crystal form is sodium salt crystal form B, and the preparation method comprises the steps of:
adding the compound of formula I to an organic solvent, adding sodium hydroxide within 1-30 minutes, stirring the reaction mixture for 5~30 minutes to induce crystallization, after crystallization, obtaining sodium salt crystal form B of the compound of Formula I, or a mixture containing a small amount of sodium salt crystal form A by filtering and drying.

In another preferred embodiment, the crystallization is selected from the group consisting of: crystallization at room temperature, cooling crystallization, solvent evaporation crystallization, seed-induced crystallization, or a combination thereof.

In another preferred embodiment, the cooling crystallization refers to crystallization at -5-25 °C.

In another preferred embodiment, the molar ratio of the compound of formula I starting material to sodium hydroxide is 1: (1-2).

In another preferred embodiment, the mass to volume ratio (g/ml) of the compound of formula I starting material to the organic solvent is 1: (20-40).

In another preferred embodiment, the sodium hydroxide is added in the form of a sodium hydroxide solution.

In another preferred embodiment, the sodium hydroxide is added in the form of solid sodium hydroxide.

In another preferred embodiment, the cooling refers to cooling at a rate of 10-20 °C/h.

In another preferred embodiment, the cooling refers to cooling to room temperature.

In another preferred embodiment, the organic solvent is selected from the group consisting of: C1-C6 alcohol solvents, C1-C6 nitrile solvents, C1-C20 sulfone solvents, C1-C20 amide solvents, C1-C6 halogenated alkane solvents, C1-C6 alkane solvents, C6-C10 aromatic hydrocarbon solvents, C2-C6 ester solvents, C1-C6 ketone solvents, C1-C6 ether solvents, or combinations thereof; preferably, C1-C6 alcohol solvent.

In another preferred embodiment, the organic solvent is selected from the group consisting of methanol, ethanol, isopropanol, acetone, tetrahydrofuran, acetonitrile, toluene, 1,4-dioxane, dichloromethane, trichloromethane, ethyl acetate, isopropyl acetate, 2-methyltetrahydrofuran, dimethyl sulfoxide, N, N-dimethylformamide, N, N-dimethylacetamide, methyl tert butyl ether, n- heptane, or combinations thereof; preferably ethanol.

In the third aspect of the present invention, provided is a pharmaceutical composition comprising (a) the sodium salt crystal form as described in the first aspect of the present invention as an active ingredient; and (b) pharmaceutically acceptable carriers.

In another preferred embodiment, the dosage form of the pharmaceutical composition is selected from the group consisting of powders, capsules, granules, tablets, pills, creams, orally disintegrating films, suspensions, or injections.

In the fourth aspect of the invention, provided is a use of the crystal form according to the first aspect of the present invention or the pharmaceutical composition according to the third aspect of the present invention for the preparation of a medicament or formulation, and the medicament or formulation is used to prevent and/or treat the diseases caused by HIV infection.

In another preferred embodiment, the dosage form of the formulation is selected from the group consisting of: powders, capsules, granules, tablets, pills, creams, orally disintegrating films, suspensions, or injections.

It should be understood that within the scope of the present invention, the above-mentioned technical features of the present invention and the specific technical features described in the following embodiments can be combined with each other to form a new or preferred technical solution. Due to space limitations, it will not be elaborated individually.

### Illustration of the drawings

Figure 1 shows the X-ray powder diffraction pattern of sodium salt crystal form A in Example 1;
Figure 2 shows the DSC&TGA spectrum of sodium salt crystal form A in Example 1;
Figure 3 shows the X-ray powder diffraction pattern of sodium salt crystal form B in Example 2;
Figure 4 shows the DSC&TGA spectrum of sodium salt crystal form B in Example 2;
Figure 5 shows the X-ray powder diffraction pattern of sodium salt crystal form A prepared in the scale-up experiment of Example 3;
Figure 6 shows the HPLC chromatogram of sodium salt crystal form A prepared in the scale-up experiment of Example 3;
Figure 7 shows the ee value spectrum of sodium salt crystal form A prepared in the scale-up experiment of Example 3;
Figure 8 shows the XRPD pattern of sodium salt crystal form A under stable conditions for 5 days in Example 6;
Figure 9 shows the XRPD pattern of sodium salt crystal form A under stable conditions for 10 days in Example 6;
Figure 10 shows the XRPD comparison of sodium salt crystal form A in Example 7 before and after DVS testing;
Figure 11 shows the DVS spectrum of sodium salt crystal form A in Example 7;
Figure 12 shows the XRPD pattern of sodium salt crystal form A after high-pressure testing in Example 8;
Figure 13 shows the XRPD comparison of sodium salt crystal form A after simulated granulation testing in Example 9.

### EMBODIMENTS FOR CARRYING OUT THE INVENTION

After a long-term and in-depth research, the inventors provided sodium salt crystal forms of an HIV integrase inhibitor molecule. The sodium salt crystal forms have advantages in stability, solubility, hygroscopicity, mechanical stability, tableting stability, flowability, process developability, formulation development, and powder processing properties, and especially the sodium salt crystal form A has significant advantages in aspects, such as stability. The inventors have completed the present invention on this basis.

### TERMS

In this article, unless otherwise specified, all abbreviations have the conventional meanings understood by those skilled in the art.

As used herein, unless otherwise specified, the solvent or solution is added by means of direct pouring or uniform (i.e., constant rate) addition, and the like.

As used herein, when referring to a specific listed value, the term "about" means that the value can vary by no more than 1% from the listed value. For example, as used herein, the expression "about 100" includes all values between 99 and 101 (e.g., 99.1, 99.2, 99.3, 99.4, etc.).

As used herein, the terms "contain" or "including(comprise)" can be open-ended, semi-closed, or closed-ended. In other words, the term also includes "essentially composed of" or "composed of".

As used herein, the term "room temperature" generally refers to 4-30 °C, preferably 20 ± 5 °C.

As used herein, the method of "slow addition" includes, but is not limited to: adding dropwise, slowly pouring along the wall of the container, etc.

### Compound of formula I

As used herein, the term "compound of formula I" refers to a compound represented by the following formula:

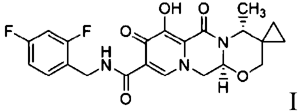

The above compound was firstly reported in patent literature CN2021113363060 of Jiangsu AIDEA Pharmaceutical Co., Ltd. and prepared by the method described therein. In this article, unless otherwise specified, the term "starting materials for the compound of formula I" refers to the compound of formula I. In the examples, API and the compound of formula I can be used interchangeably.

### Pharmaceutical composition containing sodium salt crystal form of the compound of formula I and administration mode

Since the crystal form of the present invention is a highly stable sodium salt crystal form of an HIV-1 integrase inhibitor molecule having the structure of Formula I, the sodium salt crystal form of the present invention, as well as pharmaceutical compositions containing the crystal form as the main active ingredient, can be used for the prevention and/or treatment of diseases caused by HIV infection.

The sodium salt crystal form includes sodium salt crystal form A and sodium salt crystal form B.

The pharmaceutical composition of the present invention comprises the crystal form of the present invention in a safe and effective amount range, and pharmaceutically acceptable excipients or carriers.

In which, "safe and effective amount" means that the amount of the compound is sufficient to significantly improve the condition without causing serious side effects. Generally, the pharmaceutical composition contains 1-2000 mg of the crystal form of the present invention/dose, more preferably, 10-200 mg of the crystal form of the present invention/dose. Preferably, the "one dose" is one capsule or one pill.

"Pharmaceutically acceptable carrier" means one or more compatible solid or liquid fillers, or gelatinous materials which are suitable for human use and should be of sufficient purity and sufficiently low toxicity. "Compatibility" means that each component in the composition can be admixed with the active ingredient of the present invention and with each other without significantly reducing the efficacy of the active ingredient. Examples of pharmaceutically acceptable carriers include cellulose and the derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agent (such as sodium dodecyl sulfate), coloring agents, flavoring agents, stabilizers, antioxidants, preservatives, pyrogen-free water, etc..

There is no special limitation on the administration mode for the compound or pharmaceutical compositions of the present invention, and the representative administration mode includes (but is not limited to): oral, intratumoral, rectal, parenteral (intravenous, intramuscular or subcutaneous), and topical administration.

Solid dosage forms for oral administration include capsules, tablets, pills, powders and granules. In these solid dosage forms, the active ingredient is mixed with at least one conventional inert excipient (or carrier), such as sodium citrate or dicalcium phosphate, or mixed with any of the following components: (a) fillers or compatibilizer, for example, microcrystalline cellulose, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, sodium carbonate, crospovidone, croscarmellose sodium; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants, such as talc, calcium stearate, magnesium stearate, solid polyethylene glycol, sodium lauryl sulfate, or mixtures thereof. In capsules, tablets, and pills, the dosage form may also include a buffer.

Solid dosage forms such as tablets, sugar pills, capsules, pills, and granules can be prepared using coating and shell materials, such as casings and other well-known materials in the art. They can contain an opaque agent, and the active ingredients in the composition can be released in a delayed mode in a given portion of the digestive tract. Examples of embedding components that can be used are polymeric substances and waxes. If necessary, the active ingredient can also form microcapsules with one or more of the aforementioned excipients.

Liquid dosage forms for oral administration include pharmaceutically acceptable emulsions, solutions, suspensions, syrups or tinctures. In addition to the active ingredient, the liquid dosage form may contain inert diluents conventionally used in the art, such as water or other solvents, solubilizers and emulsifiers, such as ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures of these substances.

In addition to these inert diluents, the composition can also include additives such as wetting agents, emulsifiers, suspending agents, sweeteners, corrigents, and spices.

In addition to active ingredient, suspensions can include suspending agents such as ethoxylated isooctadecanol, polyoxyethylene sorbitol and dehydrated sorbitol esters, microcrystalline cellulose, methanol aluminum and agar, or mixtures of these substances.

The compositions for parenteral injection may comprise physiologically acceptable sterile aqueous or anhydrous solutions, dispersions, suspensions or emulsions, and sterile powders for re-dissolution into sterile injectable solutions or dispersions. Suitable aqueous and non-aqueous carriers, diluents, solvents or excipients include water, ethanol, polyols, and their suitable mixtures.

The dosage forms of the crystal form of the present invention for topical administration include ointments, powders, patches, sprays, and inhalants. The active ingredients are mixed under sterile conditions with physiologically acceptable carriers and any preservatives, buffers, or propellants that may be necessary.

The crystal form of the present invention can be administered alone or in combination with other pharmaceutically acceptable compounds.

When the pharmaceutical composition is used, a safe and effective amount of the crystal form of the present invention is administered to a mammal (such as a human) in need of treatment, wherein the dosage at the time of administration is the pharmaceutically effective dosage, and for people having a body weight of 60kg, the daily dose is usually 1~2000mg, preferably 10~500mg. Of course, for the specific dosage, factors, such as the route of administration and the patient's health status, which are within the skill range of a skilled physician, should also be considered.

The pharmaceutical composition provided by the present invention preferably contains active ingredients in a weight ratio of 1-99%. The preferred ratio was that the compound of formula I accounts for 65wt% to 99wt% of the total weight as the active ingredient, and the remaining part is a pharmaceutically acceptable carrier, diluent, solution or saline solution. The compounds and pharmaceutical compositions provided by the present invention can be in various forms, such as tablets, capsules, powders, syrups, solutions, suspensions, and aerosols, and can be contained in suitable solid or liquid carriers or diluents, and in suitable disinfectants for injection or infusion.

The various dosage forms of the pharmaceutical composition of the present invention can be prepared according to conventional methods in the pharmaceutical field. The unit dosage of the formulation contains 1 mg to 700 mg of a crystal form or salt crystal form of the compound of formula I; and preferably, the unit dosage of the formulation contains 25 mg to 300 mg of the compound of formula I.

The compounds and pharmaceutical compositions of the present invention can be used clinically in mammals, including humans and animals, and can be administered via oral, nasal, cutaneous, pulmonary, or gastrointestinal routes, and the like. The most preferred option is oral administration. The most preferred daily dose is 50-1400 mg/kg body weight, administered as a single dose, or 25-700 mg/kg body weight, administered in divided doses. Regardless of the method of administration, the optimal dosage for an individual should be determined based on the specific treatment. Typically, one starts with a low dose and gradually increases the dosage until the most suitable one is found.

In the present invention, unless otherwise specified, the drying method used is a conventional drying method in the art. For example, in the embodiments of the present invention, "drying" refers to a vacuum drying or conventional pressure (atmospheric) drying in a conventional drying oven. Generally, "drying" means drying for 0.1-50 hours or 1-30 hours.

**Compared with the prior art, main advantages of the present invention include:**
(a) The crystal form of the present invention has high stability, low hygroscopicity, and high bioavailability, which is beneficial for its drug processing and use in pharmaceutical compositions.
(b) The crystal form of the present invention can be used as an active ingredient to prevent and treat diseases caused by HIV infection.

The present invention will be further explained in combination with specific examples. It should be understood that these examples are only used to illustrate the present invention and not to limit the scope of the present invention. The experimental methods without specific conditions described in the following examples are generally performed under the conventional conditions, or conditions recommended by the manufacturer. Unless otherwise stated, percentages and parts are percentages by weight and parts by weight.

### 1. Experimental materials

In the examples of the present invention, the starting material, the compound of Formula I, was a free form solid prepared according to Example 1 of AIDEA Patent literature CN2021113363060. Other reagents were commercially available industrial-grade or analytical-grade reagents.

### 2. Testing method

| | | |
|---|---|---|
| **X-ray powder diffraction** | The crystal form of the sample was analyzed using a powder X-ray diffractometer: At room temperature, the sample was ground in an agate mortar, a small amount of the sample was placed on a single crystal silicon sample plate, and it was then placed in the powder X-ray diffractometer for detection. The instrument parameters are as follows: | |
| | | 1) Instrument model: Rigaku SmartLab; |
| | | 2) X-ray emission source: Cu target; |
| | | 3) Scan range: 3~40 ° (2 θ); |
| | | 4) Scan step size: 0.02 °; |
| | | 5) Scan rate: 10 °/min. |
| **Differential Scanning Calorimetry (DSC)** | Differential Scanning Calorimetry (DSC) was used to collect data on the enthalpy changes of the sample: At room temperature, a small amount of the sample (1-3 mg) was weighed and placed in an aluminum crucible, which was then covered by cap (with a small pinhole). The sample was automatically placed onto the sample stage of the furnace by the robotic arm of the instrument for analysis. The specific instrument parameters are as follows: | |
| | | 1) Instrument model: TA 2500; |
| | | 2) Protective gas: high-purity nitrogen gas (purity>99.999%); |
| | | 3) Test range: 25~400°C; |
| | | 4) Test speed: 10°C/min. |
| **Thermogravime tric analysis (TGA)** | Thermogravimetric (TG) data of the sample was collected using a Thermogravimetric Analyzer (TGA): At room temperature, a small amount of the sample (1-3 mg) was placed in a crucible and loaded into the furnace by the autosampler of the instrument for analysis. The specific instrument parameters are as follows: | |
| | | 1) Instrument model: TA 550; |
| | | 2) Protective gas: high-purity nitrogen gas (purity>99.999%); |
| | | 3) Test range: 25~400°C; |
| | | 4) Test speed: 10°C/min. |
| **Dynamic Vapor Sorption (DVS)** | Equipment: DVS, Adventure | |
| | Humidity range: 0% -90%, step size: increments of 10% each time, relative mass per unit time dm/dt ≤ 0.2% | |
| | Temperature: 25°C ± 0.5 °C | |
| | Cycle: 2 cycles | |
| | N₂ flow rate: 200 mL/min (at 25 °C standard atmospheric pressure) | |

### Example 1: Preparation of Sodium Salt Crystal Form A

### 1. Solid-Liquid Equilibrium (SLE) experiment at 25 °C

Approximately 30 mg of the compound of formula I was weighed into each vial. Approximately 1 mL of a solvent listed in Table 1 below was added to each vial to form a suspension. Approximately 0.5 mL of an ethanol solution of sodium hydroxide (1.1 eq.) was slowly added to the suspension, which was then magnetically stirred at 25±0.1°C for 24 hours. Throughout the experiment, the sample status was observed to ensure that a suspension state was maintained; and additional solid was added if clarification occurred. The solids were isolated from all suspensions by filtration and/or centrifugation. Residual surface solvent was removed by nitrogen purging or vacuum drying. The X-ray powder diffraction pattern was shown in Figure 1, and the results were shown in Table 2. The DSC and TGA spectra were shown in Figure 2.

**Table 1 Solid-Liquid Equilibrium (SLE) experiment at 25°C**

| **Solvent** | **XRPD** |
|---|---|
| MeOH | Crystal Form A |
| EtOH | Crystal Form A |
| IPA | Crystal Form A |
| EA | Crystal Form A |
| IPAC | Crystal Form A |
| Acetone | Crystal Form A |
| THF | Crystal Form A |
| MTBE | Crystal Form A |
| ACN | Crystal Form A |
| Heptane | Crystal Form A |
| H₂O | Crystal Form A |
| DCM | Crystal Form A |
| DMSO | Crystal Form A |
| EtOH/H₂O (V/V=95/5) | Crystal Form A |
| ACN/H₂O (V/V=95/5) | Crystal Form A |

**Table 2 XRD characteristic peaks of sodium salt crystal form A**

| 2θ(°) | Intensity% |
|---|---|
| 6.308 | 1.38% |
| 7.806 | 52.40% |
| 9.107 | 92.29% |
| 11.069 | 14.46% |
| 11.686 | 14.37% |
| 12.535 | 19.17% |
| 13.120 | 8.19% |
| 13.420 | 21.78% |
| 14.807 | 6.81% |
| 15.587 | 100.00% |
| 16.332 | 24.62% |
| 16.849 | 12.61% |
| 17.994 | 19.84% |
| 18.226 | 31.47% |
| 18.622 | 26.84% |
| 19.299 | 18.76% |
| 19.525 | 16.13% |
| 19.729 | 18.33% |
| 20.828 | 39.59% |
| 21.052 | 9.67% |
| 22.016 | 16.77% |
| 22.198 | 36.15% |
| 22.472 | 4.32% |
| 23.448 | 26.33% |
| 23.712 | 16.79% |
| 24.121 | 79.71% |
| 24.425 | 4.40% |
| 24.739 | 3.15% |
| 25.241 | 18.26% |
| 25.756 | 22.64% |
| 26.147 | 27.41% |
| 26.963 | 6.32% |
| 27.738 | 38.92% |
| 28.184 | 15.74% |
| 28.907 | 12.36% |
| 29.763 | 4.84% |
| 30.262 | 5.67% |
| 30.882 | 12.71% |
| 31.392 | 4.78% |
| 31.776 | 4.62% |
| 33.217 | 10.11% |
| 34.521 | 9.64% |
| 34.820 | 3.82% |
| 35.090 | 0.68% |
| 35.908 | 2.18% |
| 36.875 | 9.30% |
| 37.645 | 2.72% |
| 38.225 | 4.39% |
| 39.014 | 0.13% |
| 39.401 | 1.75% |
| 39.689 | 3.55% |

### 2. Solid-Liquid Equilibrium (SLE) experiment at 50°C

Approximately 30 mg of the compound of formula I was weighed into each vial, and approximately 1 mL of a solvent listed in Table 3 below was added to form a suspension. Approximately 0.5 mL of an ethanol solution of sodium hydroxide (1.1 eq.) was slowly added to this suspension. The reaction mixture was stirred and heated to 50°C and held at this temperature for 2 hours. The mixture was then cooled under stirring to induce crystallization (cooling rate: 10°C/h). After cooling to 25°C, stirring was continued for an additional 5 hours. The solid was isolated to obtain sodium salt crystal form A of the compound of formula I.

**Table 3 Solid-Liquid Equilibrium (SLE) experiment at 50°C**

| **Solvent** | **XRPD** |
|---|---|
| MeOH | Crystal Form A |
| EtOH | Crystal Form A |
| IPA | Crystal Form A |
| EA | Crystal Form A |
| IPAc | Crystal Form A |
| Acetone | Crystal Form A |
| THF | Crystal Form A |
| MTBE | Crystal Form A |
| ACN | Crystal Form A |
| Heptane | Crystal Form A |
| H₂O | Crystal Form A |
| DMF | Crystal Form A |
| DMSO | Crystal Form A |
| EtOH/H₂O (V/V=95/5) | Crystal Form A |
| ACN/H₂O (V/V=95/5) | Crystal Form A |

### Example 2: Preparation of Sodium Salt Crystal Form B

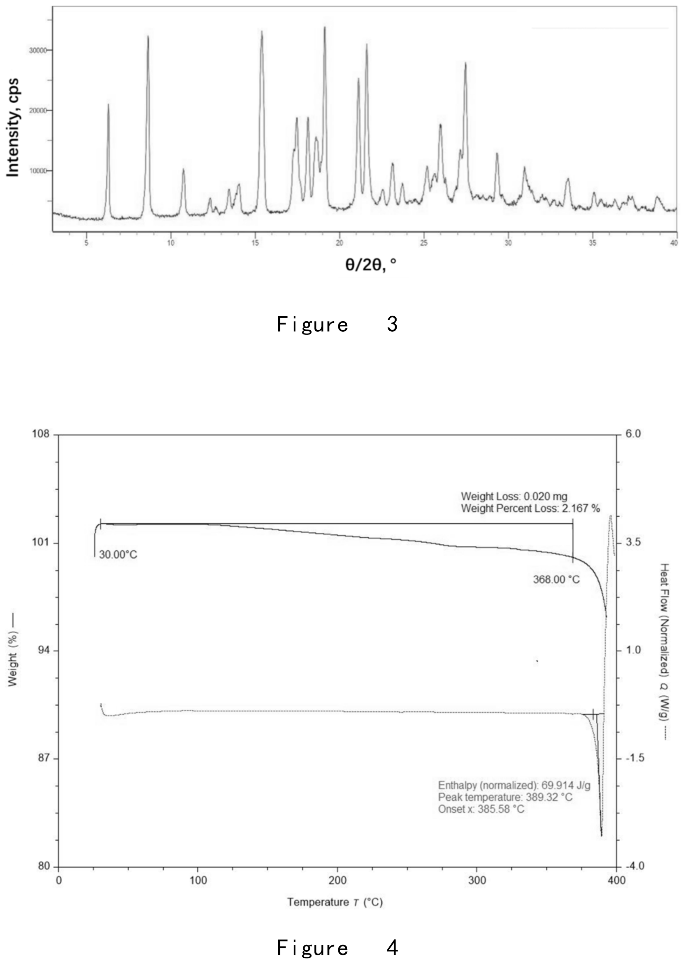

Approximately 100 mg of the compound of Formula I was weighed and added into approximately 0.5 mL of ethanol to obtain a suspension. Approximately 0.5 mL of an ethanol solution of sodium hydroxide (2.0 eq.) was slowly added to this suspension over 1-30 minutes. The mixture was stirred at room temperature for about 5-10 minutes, and the solid was isolated to obtain sodium salt crystal form B of the compound of formula I. The X-ray powder diffraction pattern was shown in Figure 3, and the results were shown in Table 4. The DSC and TGA spectra were shown in Figure 4.

**Table 4 XRD characteristic peaks of sodium salt crystal form B**

| 2θ(°) | Intensity % |
|---|---|
| 6.3220 | 34.82% |
| 8.673 | 76.09% |
| 10.769 | 22.99% |
| 12.328 | 6.10% |
| 12.672 | 2.85% |
| 13.481 | 9.65% |
| 14.046 | 22.65% |
| 15.384 | 100.00% |
| 17.07 | 2.20% |
| 17.267 | 29.79% |
| 17.468 | 40.09% |
| 18.098 | 37.19% |
| 18.606 | 58.55% |
| 19.097 | 82.71% |
| 21.115 | 64.07% |
| 21.609 | 75.71% |
| 22.543 | 6.82% |
| 23.132 | 19.25% |
| 23.716 | 7.54% |
| 25.180 | 17.12% |
| 25.980 | 82.91% |
| 27.180 | 45.71% |
| 27.442 | 51.61% |
| 29.323 | 20.86% |
| 29.620 | 2.42% |
| 30.64 | 4.01% |
| 30.947 | 29.92% |
| 31.99 | 3.54% |
| 33.50 | 14.94% |
| 35.05 | 6.39% |
| 35.49 | 4.04% |
| 36.322 | 3.83% |
| 36.75 | 2.04% |
| 37.29 | 11.28% |
| 38.788 | 9.27% |

### Example 3: Scale-up experiment of sodium salt crystal form A

52.6 kg of anhydrous ethanol, 6.6 kg of purified water, and 2.445 kg of the compound of formula I were added to reactor R1, and stirring was initiated. The temperature was raised to 68-78 °C, and the mixture was stirred to obtain a clear solution. The solution was then pressure-filtered through a precision filter into reactor R2 (located in the clean area), where it was again heated to 68-78°C and stirred until a clear solution was obtained.

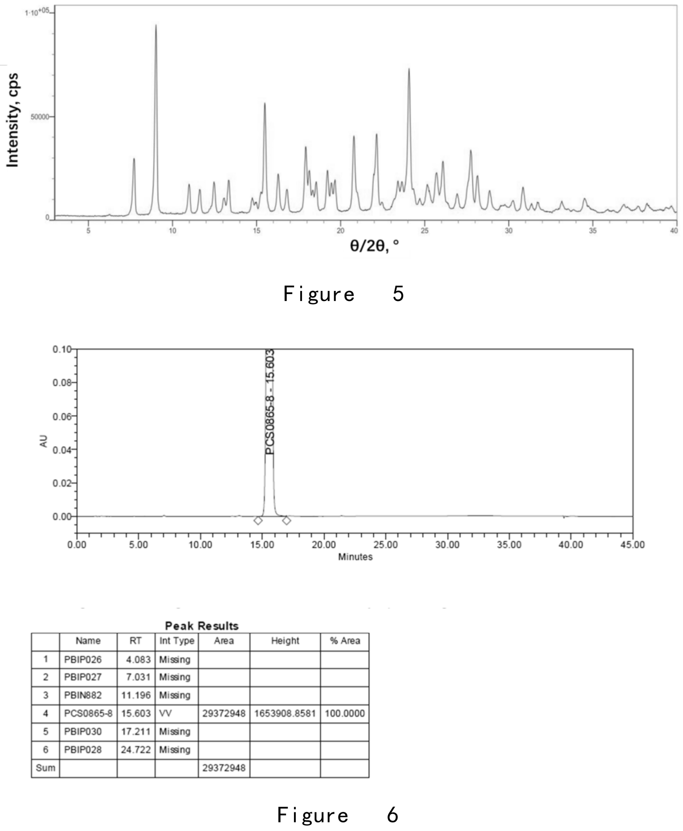

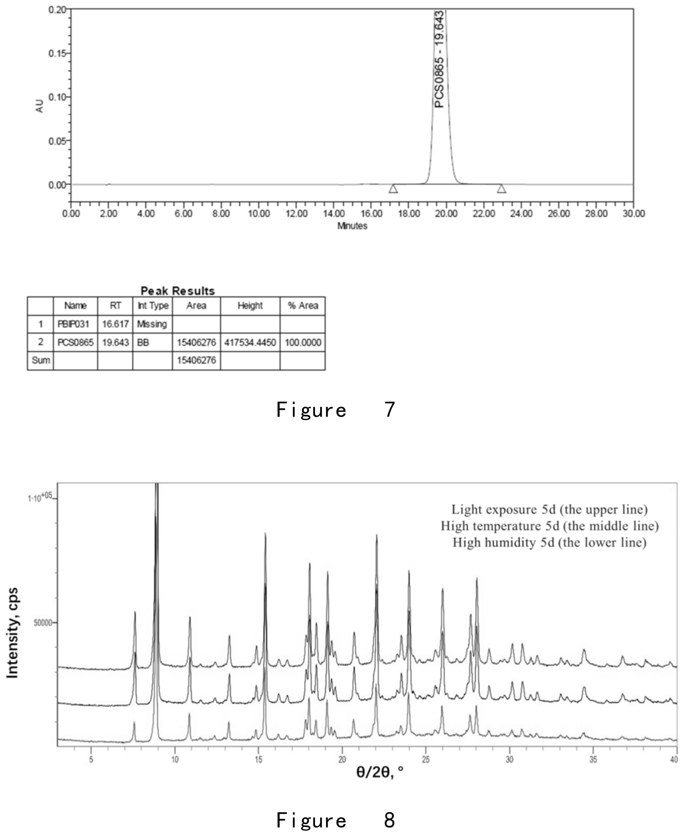

A solution of 0.295 kg of sodium hydroxide and 1.61 kg of purified water was prepared separately and pressure-filtered through a precision filter into the addition tank of reactor R2, located in the clean area. After 60 g of sodium salt crystal form A seeds was added to reactor R2, the temperature was controlled at 68-78°C and the sodium hydroxide solution was slowly added dropwise. Upon the addition, the reaction was held at a constant temperature and stirred for 22 hours. The reaction mixture was then slowly cooled to 10-15°C and continuously stirred for 3 hours to induce crystallization. The mixture was centrifuged, and the wet product was washed with ethanol. The wet product was transferred to a dryer, with the jacket temperature controlled at 45-55°C, and dried under vacuum to yield 2.54 kg of sodium salt crystal form A. (Yield 96.7%, purity 100%, ee value 100%, moisture 0.3%, residual ethanol 492ppm). The X-ray powder diffraction pattern was shown in Figure 5, the HPLC spectrum was shown in Figure 6, and the ee value spectrum was shown in Figure 7.

### Example 4: Physical and Chemical Properties of Sodium Salt Crystal Form A and Crystal Form B

The sodium salt crystal form A and crystal form B prepared in Example 1 and Example 2, respectively, were subjected to DSC, TGA, and HPLC tests. The data results are shown in Table 5.

**Table 5 Physical and Chemical Properties of Sodium Salt Crystal Form A and Crystal Form B**

| **Polymorphs** | **Crystal Form A** | **Crystal Form B** |
|---|---|---|
| Crystallinity | High | High |
| DSC melting point (° C) | 394.96 | 389.32 |
| TGA weight loss | 0.367% at 380°C | 2.167% at 368°C |
| Form | Fine particles | Fine particles |
| Purity (%) | 99.6% | 99.5% |

Both sodium salt crystal forms have high melting points, good hygroscopicity and solid-state stability, and excellent crystallinity.

### Example 5: Solubility Test

5-50 mg each of the compound of formula I and sodium salt crystal form A were suspended in 2-10 mL of solvent in glass vials, respectively. After stirring the suspensions at 25°C for 48 h, the suspensions were filtered. The solubility was calculated by determining the concentration of the saturated solution via HPLC. The results were shown in Table 6.

**Table 6**

| **Sample** | **Solvent** | **Solubility at 25 °C (µg/mL)** |
|---|---|---|
| Compound of formula I | Water | 5.1 |
| Sodium salt crystal form A | Water | 500.2 |

The results showed that at 25 °C, the solubility of sodium salt crystal form A in water was significantly higher than that of compound I.

### Example 6: Factors affecting sodium salt crystal form A

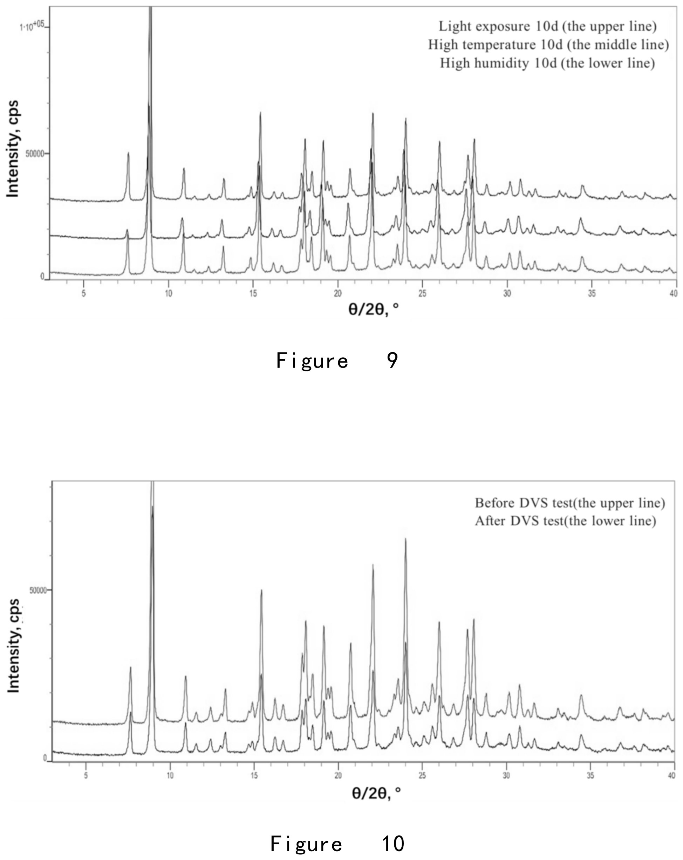

70 mg samples of sodium salt crystal form A were accurately weighed and placed into individual glass petri dishes. The petri dishes were then transferred into constant temperature and humidity chambers under three different conditions: high temperature (60 °C, sealed), high relative humidity (RH: 90 ± 5%, 25 °C, open), and lighting (5000 ± 500 Lux, open). The samples were tested on Day 5 and Day 10. The samples were analyzed by XRPD and HPLC, as shown in Figures 8 and 9, and the color change of the samples was observed. The results were shown in Table 7.

**Table 7 Experiment on factors affecting sodium salt crystal form A**

| **Examination conditions** | **XRPD** | **Purity** | **Property** |
|---|---|---|---|
| **Day 0** | | | |
| Sodium salt crystal form | Crystal Form A | 99.57% | off-white solid |

| **Day 5** | | | |
|---|---|---|---|
| Lighting | Crystal Form A | 99.68% | off-white solid |
| High temperature (60 °C) | Crystal Form A | 99.69% | off-white solid |

| **Examination conditions** | **XRPD** | **Purity** | **Property** |
|---|---|---|---|
| High humidity (90 ± 5%) | Crystal Form A | 99.64% | off-white solid |

| **Day 10** | | | |
|---|---|---|---|
| Lighting | Crystal Form A | 99.72% | off-white solid |
| High temperature (60 °C) | Crystal Form A | 99.72% | off-white solid |
| High humidity (90 ± 5%) | Crystal Form A | 99.72% | off-white solid |

The above results indicate that: under high temperature, high humidity, and light exposure conditions, the properties of sodium salt crystal form A did not change, the HPLC purity showed minimal change, and the XRPD did not change. This demonstrated that sodium salt crystal form A exhibited good physicochemical stability.

### Example 7: Hygroscopicity of Sodium Salt Crystal Form A

20 mg of the sodium salt crystal form A sample was weighed for DVS testing. The sample, after DVS testing, was then analyzed by XRPD. The results were shown in Table 8 below.

**Table 8 DVS test results of sodium salts**

| Relative humidity | **Adsorption (%)** | **Desorption (%)** | **Adsorption (%)** | **Desorption (%)** |
|---|---|---|---|---|
| **0%** | 0.0000 | -0.0471 | -0.0471 | -0.0614 |
| **10%** | 0.0668 | 0.0285 | 0.0230 | 0.0147 |
| **20%** | 0.1197 | 0.0891 | 0.0818 | 0.0758 |
| **30%** | 0.1725 | 0.1498 | 0.1394 | 0.1373 |
| **40%** | 0.2282 | 0.2239 | 0.2001 | 0.2149 |
| **50%** | 0.2857 | 0.3320 | 0.2641 | 0.3200 |
| **60%** | 0.3539 | 0.5250 | 0.3365 | 0.5185 |
| **70%** | 0.5505 | 0.6126 | 0.5627 | 0.6037 |
| **80%** | 0.6895 | 0.7176 | 0.6928 | 0.7082 |
| **90%** | 0.9012 | 0.9012 | 0.8889 | 0.8889 |
| **After DVS testing Crystal form change** | Crystal form A, unchanged | | | |

As shown in Figures 10 and 11, the DVS test results for two times for sodium salt crystal form A showed: the weight gain of sodium salt crystal form A at 80% relative humidity (RH) was 0.69%, indicating that crystal form A has low hygroscopicity. The crystal form of the sample did not change before and after DVS testing, demonstrating that crystal form A possesses high humidity stability.

### Example 8: High pressure property test of sodium salt crystal form A

For the high-pressure property test, approximately 300 mg of the sodium salt crystal form A sample was weighed and loaded into a hydraulic die with a diameter of 13 mm and pressed using a manual hydraulic press at 10 MPa for 5 minutes. The resulting solid was characterized by XRPD, as shown in Figure 12. After the high-pressure test, sodium salt crystal form A did not change.

### Example 9: Sodium salt crystal form A simulated granulation test

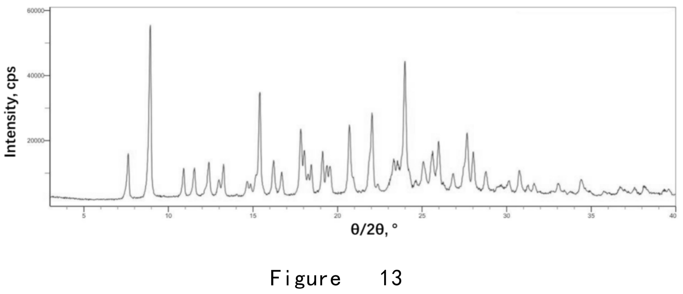

Ethanol was added dropwise to the sodium salt crystal form A sample until the solid was sufficiently wetted, and it was then ground. The resulting solid and the sample without the addition of wetting ethanol were analyzed by XRPD, respectively. The results were shown in Figure 13. The results showed that sodium salt crystal form A, after ethanol wetting and grinding, did not exhibit a change in crystal form, indicating that crystal form A possesses granulation stability.

### Example 10: Pharmacokinetic Study

Sodium salt crystal form A, sodium salt crystal form B, and the compound of formula I (formulated as a 0.5% HPMC/0.1% Tween-80 suspension) were orally administered to male SD rats (a dose of 5 mg/kg and a dosing volume of 10 mL/kg, n=3). Blood samples (50 µL) were collected via jugular vein puncture at pre-dose and at 0.25, 0.5, 1, 2, 4, 8, 10, and 24 hours post-dose. The samples were placed into centrifuge tubes containing an anticoagulant (EDTA-K2), mixed by inverting, and placed on crushed ice. Within 2 hours, the samples were centrifuged at 3500 rpm for 10 minutes at 4°C. The plasma was collected and transferred to the corresponding labeled EP tube, and stored in a -80 °C ultra-low temperature freezer until analysis and detection. Prior to analysis, the plasma in the EP tubes was thawed at room temperature and vortex-mixed. A 10 µL aliquot of the sample was taken for LC-MS/MS determination of the compound of Formula I concentration in the plasma. Plasma concentration data and pharmacokinetic parameters were calculated and summarized using WinNonlin 8.1, as shown in Table 9.

**Table 9 Results of pharmacokinetic parameters**

| PK parameters Mean±SD | Sodium salt crystal form A (n=3) | Sodium salt crystal form B (n=3) | Compound of Formula 1 (n=3) |
|---|---|---|---|
| Cₘₐₓ (ng/mL) | 9443 ± 470 | 8703 ± 490 | 7977 ± 2187 |
| AUC₀₋ₜ (ng·hr/mL) | 138559 ±17078 | 119171 ± 7203 | 109798 ± 23693 |
| AUC_{0-∞} (ng·hr/mL) | 189762 ± 33837 | 159326 ± 8305 | 152906 ± 23562 |

The above results indicate that, compared to sodium salt crystal form B and the compound of formula I, sodium salt crystal form A of the present invention has higher plasma Cₘₐₓ and AUC values of the compound of formula I following oral administration to rats. This demonstrates higher plasma exposure of the compound of formula I from crystal form A under identical dosing conditions, suggesting that sodium salt crystal form A may possess superior biological absorption.

All documents mentioned in the present invention are cited as references in this application, just as each document is individually cited as a reference. In addition, it should be understood that, after reading the above teaching content of the present invention, those skilled in the art can make various changes or modifications to the present invention, and these equivalent forms also fall within the scope defined by the appended claims of the present application.

## Claims

1. A sodium salt crystal form of a compound of formula I, wherein 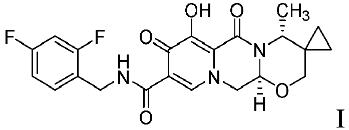
the sodium salt crystal form of the compound of formula I is sodium salt crystal form A, and the sodium salt crystal form A has the following X-ray powder diffraction characteristic peaks (2 θ angle): 15.587 °± 0.2 °, 9.107 °± 0.2 °, 24.121 °± 0.2 °, 7.806 °± 0.2 °, 20.828 °± 0.2 °; or
the sodium salt crystal form of the compound of formula I is sodium salt crystal form B, and the sodium salt crystal form B has the following X-ray powder diffraction characteristic peaks (2 θ angle): 15.384 °± 0.2 °, 25.98 °± 0.2 °, 19.097 °± 0.2 °, 8.673 °± 0.2 °, 21.609 °± 0.2 °.

2. The crystal form according to claim 1, wherein,
the X-ray powder diffraction pattern of the sodium salt crystal form A further includes 2 or more (3, 4, or 5) X-ray powder diffraction characteristic peaks (2 θ angles) selected from the group consisting of: 27.738 °± 0.2 °, 22.198 °± 0.2 °, 18.226 °± 0.2 °, 26.147 °± 0.2 °, 18.622 °± 0.2 °, 23.448 °± 0.2 °; or
the X-ray powder diffraction pattern of the sodium salt crystal form B further includes 2 or more (3, 4, or 5) X-ray powder diffraction characteristic peaks (2 θ angles) selected from the group consisting of: 21.115 °± 0.2 °, 18.606 °± 0.2 °, 27.442 °± 0.2 °, 27.18 °± 0.2 °, 17.468 °± 0.2 °, 18.098 °± 0.2 °.

3. The crystal form according to claim 2, wherein the sodium salt crystal form A further has one or more X-ray powder diffraction characteristic peaks (2 θ angles) selected from the group consisting of: 16.332 °± 0.2 °, 25.756 °± 0.2 °, 13.420 °± 0.2 °, 17.994 °± 0.2 °, 12.535 °± 0.2 °, 19.299 °± 0.2 °, 19.729 °± 0.2 °, 25.241 °± 0.2 °, 23.712 °± 0.2 °; or
the sodium salt crystal form B further has one or more X-ray powder diffraction characteristic peaks (2 θ angles) selected from the group consisting of: 6.322 °± 0.2 °, 30.947 °± 0.2 °, 17.267 °± 0.2 °, 10.769 °± 0.2 °, 14.046 °± 0.2 °, 29.323 °± 0.2 °, 23.132 °± 0.2 °, 25.18 °± 0.2 °, 33.5 °± 0.2 °.

4. The crystal form according to claim 3, wherein the sodium salt crystal form A has X-ray powder diffraction characteristic peaks (2 θ angles) selected from the group consisting of: 22.016 °± 0.2 °, 19.525 °± 0.2 °, 28.184 °± 0.2 °, 11.069 °± 0.2 °, 11.686 °± 0.2 °, 30.882 °± 0.2 °, 16.849 °± 0.2 °, 28.907 °± 0.2 °, 33.217 °± 0.2 °, 21.052 °± 0.2 °, 34.521 °± 0.2 °, 36.875 °± 0.2 °, 13.120 °± 0.2 °, 14.807 °± 0.2 °, 26.963 °± 0.2 °, 30.262 ° ± 0.2 °, 29.763 °± 0.2 °, 31.392 °± 0.2 °, 31.776 °± 0.2 °, 24.425 °± 0.2 °, 38.225 °± 0.2 °, 22.472 °± 0.2 °, 34.820 °± 0.2 °, 39.689 °± 0.2 °, 24.739 °± 0.2 °, 37.645 °± 0.2 °, 35.908 °± 0.2 °, 39.401 °± 0.2 °, 6.308 °± 0.2 °, 35.090 °± 0.2 °, 39.014 °± 0.2 °; or
the sodium salt crystal form B has X-ray powder diffraction characteristic peaks (2 θ angles) selected from the group consisting of: 37.29 °± 0.2 °, 13.481 °± 0.2 °, 38.788 °± 0.2 °, 23.716 °± 0.2 °, 22.543 °± 0.2 °, 35.05 °± 0.2 °, 12.328 °± 0.2 °, 35.49 °± 0.2 °, 30.64 °± 0.2 °, 36.322 °± 0.2 °, 31.99 °± 0.2 °, 12.672 °± 0.2 °, 29.62 °± 0.2 °, 17.07 °± 0.2 °, 36.75 °± 0.2 °.

5. The crystal form according to claim 1, wherein the thermogravimetric analysis (TGA) thermogram of the sodium salt crystal form A shows a weight loss of 0.2%~2.0% in the range of 100 °C~380 °C; or the differential scanning calorimetry of the sodium salt crystal form A shows an endothermic peak at 385 °C~395 °C.

6. The crystal form according to claim 1, wherein the sodium salt crystal form A further has one or more of the following characteristics (1) - (4):
(1) The sodium salt crystal form A has XRPD data substantially as shown in Table 1; and/or
(2) The sodium salt crystal form A has an XRPD spectrum substantially as shown in Figure 1; and/or
(3) The sodium salt crystal form A has a DSC&TGA spectrum substantially as shown in Figure 2; and/or
(4) The sodium salt crystal form A has a DVS spectrum substantially as shown in Figure 11.

7. A method for preparing the crystal form according to claim 1, wherein the method comprises the steps of:
adding the compound of formula I to an organic solvent or an aqueous organic solvent, reacting with sodium hydroxide, and obtaining the sodium salt crystal form by crystallization.

8. The method according to claim 7, wherein the organic solvent is selected from the group consisting of: C1-C6 alcohol solvents, C1-C6 nitrile solvents, C1-C20 sulfone solvents, C1-C20 amide solvents, C1-C6 halogenated alkane solvents, C1-C6 alkane solvents, C6-C10 aromatic hydrocarbon solvents, C2-C6 ester solvents, C1-C6 ketone solvents, C1-C6 ether solvents, or combinations thereof; preferably, C1-C6 alcohol solvent.

9. A pharmaceutical composition comprising (a) the sodium salt crystal form according to claim 1 as an active ingredient; and (b) pharmaceutically acceptable carriers.

10. Use of the crystal form according to claim 1 or the pharmaceutical composition according to claim 9 in the preparation of a medicament or formulation for the prevention and/or treatment of diseases caused by HIV infection.
